# EUROPEAN PATENT APPLICATION

(11) **EP 3 885 869 A1**
(43) Date of publication of application: **29.09.2021**
(21) Application number: 21152546.4
(22) Date of filing: 20.01.2021
(51) Int. Cl.: G05D 1/02, G05D 1/10, G05D 1/00

(54) **SYSTEM AND METHOD FOR TRACKING A HUMAN TARGET**

(30) Priority: 27.03.2020 US 202016832975
(71) Applicant: NXP B.V., 5656 AG Eindhoven (NL)
(72) Inventor: RAJAN KESAVELU SHEKAR, Pramod, Redhill, Surrey RH1 1QZ (GB); SHIRWAL, Anand, Redhill, Surrey RH1 1QZ (GB)
(74) Representative: Hardingham, Christopher Mark

(57) **Abstract**

A system includes an electronic device for placement on a target. The electronic device includes first and second wearable structures physically displaced away from one another and having corresponding first and second communication modules.
The system further includes a drone for monitoring motion of the target. The drone includes a third communication module. The first and third communication modules enable a first wireless communication link between the first wearable structure and the drone, and the second and third communication modules enable a second wireless communication link between the second wearable structure and the drone.
The drone further includes a processing unit for determining a current location of the drone relative to the target using the first and second wireless communication links and a drive control unit for adjusting a speed and a position of the drone relative to the target.

## Description

### TECHNICAL FIELD OF THE INVENTION

The present invention relates generally to systems and methodology for monitoring a moving target. More specifically, the present invention relates to real time autonomous positioning and navigation of an unmanned vehicle relative to a moving target for monitoring and analyzing the motion of the moving target.

### BACKGROUND OF THE INVENTION

In sport and exercise, sports biomechanics is a quantitative based study and analysis of athletes and sports activities in general. Thus, sports biomechanics refers to the study of human movements, including the interaction between the athlete, sport equipment, and the exercise environment in order to gain a greater understanding of athletic performance for the purposes of enhancing athletic performance, minimizing injuries, promoting career longevity, and so forth.

### SUMMARY

Aspects of the disclosure are defined in the accompanying claims.

In a first aspect, there is provided a system comprising an electronic device comprising a first wearable structure configured to be positioned on a target, the first wearable structure including a first communication module; and a second wearable structure configured to be positioned on the target, the second wearable structure being physically displaced away from the first wearable structure, the second wearable structure including a second communication module; and the system further comprising an unmanned vehicle for monitoring motion of the target, the unmanned vehicle comprising a third communication module, wherein the first and third communication modules are configured to enable a first wireless communication link between the first wearable structure and the unmanned vehicle, and the second and third communication modules are configured to enable a second wireless communication link between the second wearable structure and the unmanned vehicle; a processing unit configured to determine a current location of the unmanned vehicle relative to the target in response to the first and second wireless communication links; and a drive control unit in communication with the processing unit and configured to adjust a speed and a position of the unmanned vehicle to move the unmanned vehicle from the current location to a predefined location relative to the target.

In a second aspect, there is provided a method utilizing an unmanned vehicle for monitoring motion of a target comprising positioning first and second wearable structures of an electronic device on the target, the first and second wearable structures being physically displaced away from one another, the first wearable structure including a first communication module, and the second wearable structure including a second communication module; enabling a first wireless communication link between the first communication module of the first wearable structure and a third communication module on-board the unmanned vehicle; enabling a second wireless communication link between the second communication module of the second wearable structure and the third communication module; determining a current location of the unmanned vehicle relative to the target in response to the first and second wireless communication links; and adjusting a speed and a position of the unmanned vehicle to move the unmanned vehicle from the current location to a predefined location relative to the target.

### BRIEF DESCRIPTION OF THE DRAWINGS

The accompanying figures in which like reference numerals refer to identical or functionally similar elements throughout the separate views, the figures are not necessarily drawn to scale, and which together with the detailed description below are incorporated in and form part of the specification, serve to further illustrate various embodiments and to explain various principles and advantages all in accordance with the present invention.
FIG. 1 shows an example of a system that includes an electronic device worn by a target (e.g., a human user) and an unmanned vehicle;
FIG. 2 shows a front view of the human user wearing the electronic device;
FIG. 3 shows a block diagram of the electronic device worn by the human user;
FIG. 4 shows a simplified block diagram of components on-board the unmanned vehicle;
FIG. 5 shows a flowchart of a target monitoring and motion analysis process performed using the system of FIG. 1;
FIG. 6 shows a flowchart of an adaptive speed and position control subprocess of the target monitoring and motion analysis process of FIG. 5;
FIG. 7 shows a flowchart of a data acquisition subprocess of the target monitoring and motion analysis process of FIG. 5;
FIG. 8 shows a flowchart of a motion analysis subprocess of the target monitoring and motion analysis process of FIG. 5; and
FIG. 9 shows a flowchart of a feedback provision subprocess of the target monitoring and motion analysis process of FIG. 5.

### DETAILED DESCRIPTION

In overview, the present disclosure concerns a system and methodology for monitoring motion of a target, such as a human user. More particularly, the system and methodology entail real time autonomous positioning and navigation of an unmanned vehicle relative to the moving target. The unmanned vehicle and an electronic device positioned on the target communicate to locate the target and position the unmanned vehicle relative to the target. The unmanned vehicle includes a sensor system (e.g., a camera) for capturing motion of the moving target. In some embodiments, the electronic device positioned on the target also includes a sensor system (e.g., motion/pressure sensors, vitals monitors, and so forth) configured to detect physiological indicators of the target. The unmanned vehicle includes a processing unit configured to adjust the position of the unmanned vehicle relative to the moving target, control the on-board sensor system (e.g., camera), receive visual information of the motion of the target, receive the physiological indicators of the target, analyze the motion of the target (e.g., human user) based on the visual information alone or in combination with the physiological indicators, and provide real time feedback to the human user regarding the motion analysis. In some embodiments, a human user can provide voice commands via the electronic device to control the unmanned vehicle.

The description provided below relates to monitoring motion of a human user utilizing an unmanned aerial vehicle, commonly known as a drone, for the purpose of gait analysis. It should be appreciated, however, that embodiments described below may be generalized to other targets to be monitored, such as other animate or inanimate objects. Thus, gait analysis may be performed for another animate object (e.g., animal), or motion analysis may be performed for an inanimate object (e.g., vehicle, shipping container, and the like) for tracking purposes.

The instant disclosure is provided to further explain in an enabling fashion at least one embodiment in accordance with the present invention. The disclosure is further offered to enhance an understanding and appreciation for the inventive principles and advantages thereof, rather than to limit in any manner the invention. The invention is defined solely by the appended claims including any amendments made during the pendency of this application and all equivalents of those claims as issued.

It should be understood that the use of relational terms, if any, such as first and second, top and bottom, and the like are used solely to distinguish one from another entity or action without necessarily requiring or implying any actual such relationship or order between such entities or actions.

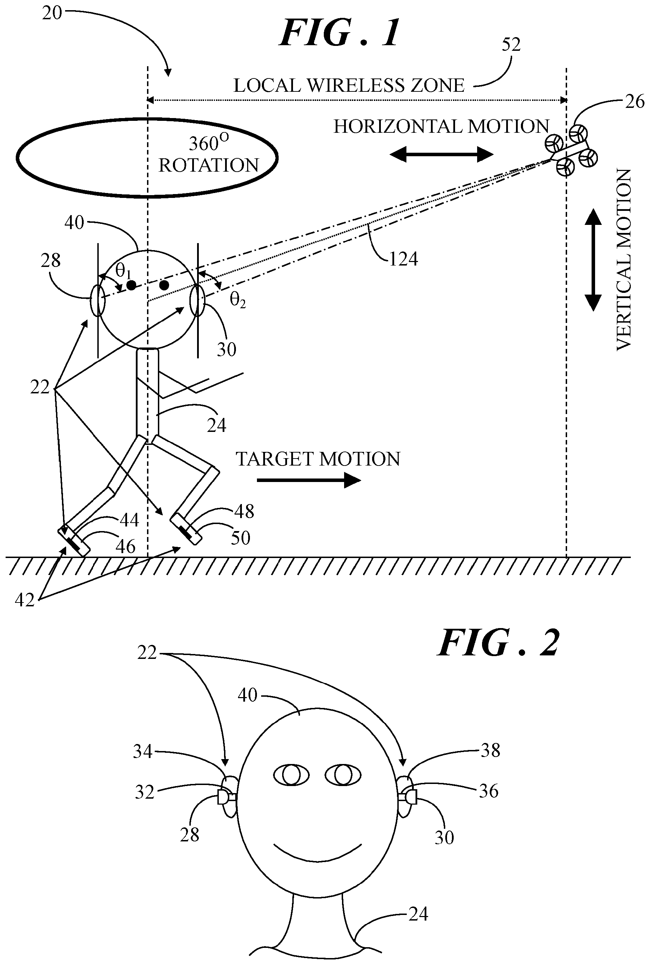

Referring to FIGs. 1 and 2, FIG. 1 shows an example of a system 20 that includes an electronic device 22 worn by a target 24 and an unmanned vehicle 26 and FIG. 2 shows a front view of the target 24 wearing electronic device 22. As discussed herein, target 24 is a human user. As such, target 24 will be generally referred to herein as a user 24. Unmanned vehicle 26 may be any of a number of vehicles including, for example, unmanned aerial vehicles (UAV), unpiloted aerial vehicles, remotely piloted aircraft, unmanned aircraft systems, any aircraft covered under Circular 328 AN/190 classified by the International Civil Aviation Organization, and so forth. As an example, unmanned vehicle 26 may be in the form or a single or multi-rotor copter (e.g., a quadcopter) or a fixed wing aircraft. In addition, certain aspects of the disclosure may be utilized with other types of unmanned vehicles (e.g., wheeled, tracked, spacecraft, and/or water vehicles). For simplicity, unmanned vehicle 26 will be generally referred to herein as a drone 26.

Electronic device 22 of system 20 includes first and second wearable structures 28, 30 configured to be positioned on user 24, with second wearable structure 30 being physically displaced away from first wearable structure 28. As best shown in FIG. 2, first wearable structure 28 includes at least a first portion 32 configured to be disposed within a first ear 34 of the human user 24 and second wearable structure 30 includes a second portion 36 configured to be disposed with a second ear 38 of human user 24. The wear location of first and second wearable structures 28, 30 places each of them in a near constant position and orientation with respect to the head 40/ears 34, 38 of user 24.

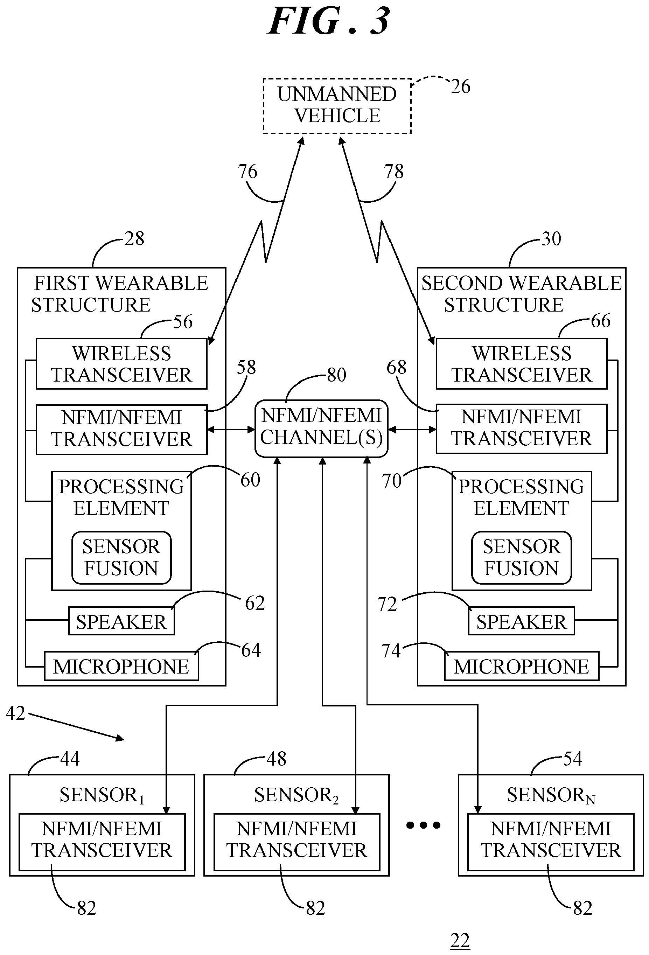

In the example embodiment, first and second wearable structures 28, 30 may be hearing instruments, sometimes simply referred to as hearables. In this instance, first and second wearable structures 28, 30 as hearables may include a microphone and speaker combination, a processing element to process the signal captured by the microphone and to control the output of the speaker, and one or more wireless communication modules (e.g., transceivers) for enabling wireless communication. Further details of the components within first and second wearable structures 28, 30 will be provided below in connection with FIG. 3. In alternative embodiments, first and second wearable structures 28, 30 need not be hearables, but may be any suitable electronic device that can be positioned on the target for the purpose of monitoring and analyzing motion of the target.

Electronic device 22 may additionally include a sensor system 42 positioned on user 24. Sensor system 42 is configured to sense at least one indicator, referred to herein as a physiological indicator, of user 24. In the example embodiment, sensor system 42 may include a first body sensor 44 (represented by a dark rectangle) coupled to a first foot 46 of user 24 and a second body sensor 48 (represented by another dark rectangle) coupled to a second foot 50 of user 24. First and second body sensors 44, 48 represent a wide variety of sensing elements such as, for example, motion sensors (e.g., accelerometers, gyroscopes, magnetometers, inertial sensors, pressure sensors and the like) that may be strapped, adhered, or otherwise coupled to first and second feet 46, 50. Additional and/or alternative sensors may be encompassed within sensor system 42. These additional and/or alternative sensors need not be coupled to feet 46, 50, but may instead have a separate attachment to user 24 or may be housed within either of first and second wearable structures 28, 30. These other sensors may entail an oxygen sensor, heart rate sensor, blood pressure sensor, pedometer, distance measuring unit, or any other suitable sensor.

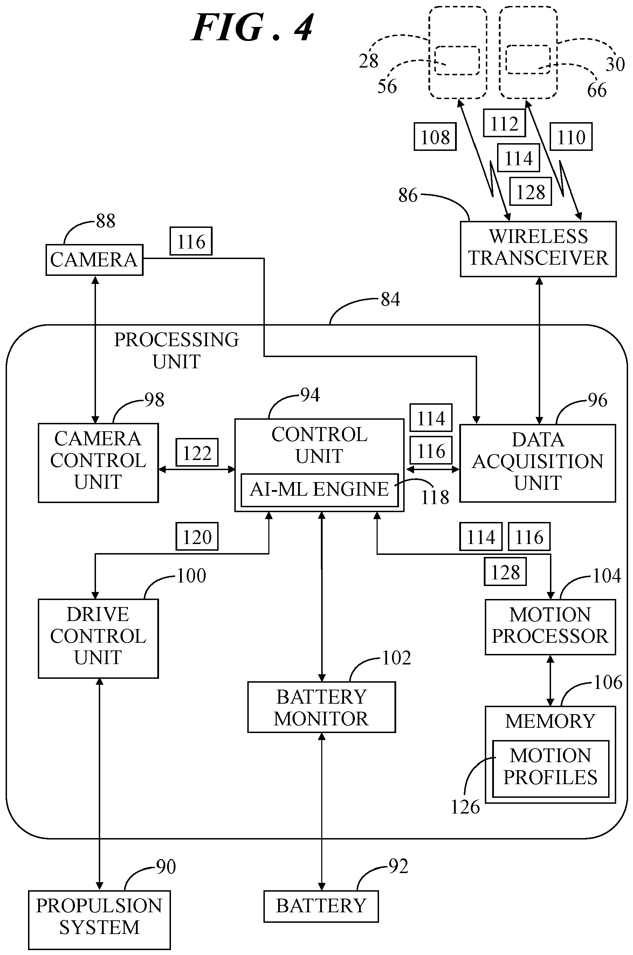

Embodiments entail real time autonomous positioning and navigation of drone 26 relative to user 24 for the purpose of data collection of motion information of user 24, data analysis of the motion information, and feedback of the analyzed motion information to user 24 so that user 24 may take corrective action. As will be discussed in significantly greater detail below, drone 26 and electronic device 22 are configured to cooperatively establish a local wireless communication zone 52 so as to enable communication between electronic device and drone 26 for at least autonomous positioning and navigation of drone 26 relative to user 24 (vertical/horizontal motion, 360° rotation about user 24, and adaptive speed based upon target motion), data communication, feedback, voice commands, gesture commands, and so forth. Further details of the components within drone 26 will be provided below in connection with FIG. 4.

FIG. 3 shows a block diagram of electronic device 22 worn by human user 24 (FIG. 1). As mentioned previously electronic device 22 includes first wearable structure 28, second wearable structure 30, and sensor system 42. Sensor system 42 may include any variety and quantity of suitable sensors, as discussed above. As such, sensor system 42 is shown as including first body sensor 44 (SENSOR₁), second body sensor 48, (SENSOR₂), and additional sensors 54 (SENSOR_{N}) separated by ellipses from second body sensor 48 to indicate any quantity "N" of sensors.

First wearable structure 28 includes at least a first communication module 56 (WIRELESS TRANSCEIVER), a first near field magnetic induction/near field electromagnetic induction (NFMI/NFEMI) transceiver 58, and a processing element 60. In some embodiments, first wearable structure 28 may additionally include a speaker 62 and a microphone 64. Similarly, second wearable structure 30 includes at least a second communication module 66 (WIRELESS TRANSCEIVER), a second NFMI transceiver 68, and a processing element 70. In some embodiments, second wearable structure 30 may additionally include a speaker 72 and a microphone 74. NFMI refers to a short-range communication technique that makes use of transmissions within a localized magnetic field. NFEMI, which is an extension of NFMI, is a communication technique that also makes use of transmissions within a localized magnetic field and uses an electric antenna for transmissions.

In general, first communication module 56 of first wearable structure 28 is configured for communication with drone 26 via a first wireless communication link 76 and second communication module 66 of second wearable structure 30 is configured for communication with drone 26 via a second first wireless communication link 78. Additionally, first and second NFMI transceivers 58, 68 enable wireless communication (generally represented by NFMI CHANNELS 80) between first and second wearable structures 28 and between NFMI transceivers 82 associated with each of the various sensors 44, 48, 54 of sensor system 42. As will be discussed in greater detail below, a first wireless communication technology (e.g., Bluetooth communication) is implemented to enable communication via first and second communication links 76, 78 and thereby establish local wireless zone 52 (FIG. 1). A second wireless communication technology (e.g., near-field magnetic induction communication) is implemented to enable communication between first and second wearables 28, 30 and between NFMI transceivers 82 associated with each of the various sensors 44, 48, 54 of sensor system 42.

FIG. 4 shows a simplified block diagram of components on-board drone 26. In general, drone 26 includes a processing unit 84, third communication module 86 (WIRELESS TRANSCEIVER), a sensor system in the form of a camera 88, and a propulsion system 90 (e.g., one or more motors), all of which are powered by a battery 92. Processing unit 84 can include a control unit 94, a data acquisition unit 96, a camera control unit 98, a drive control unit 100, battery monitor circuit 102 (monitoring a battery output voltage), a motion processor 104, and a memory element 106. One or more communication buses, such as a CAN bus, or signal lines may couple the components of processing unit 84, third communication module 86, camera 88, propulsion system 90, and battery 92.

Third communication module 86 residing on drone 26 is configured to communicate with first and second wearable structures 28, 30. More particularly, first and third communication modules 56, 86 are configured to enable and maintain first wireless communication link 76 and second and third communication modules 66, 86 are configured to enable and maintain second wireless communication link 78. In general, first and second location data 108, 110 may be communicated via respective first and second communication links 76, 78 and may be utilized to adjust the speed and position of drone 26 relative to user 24 (FIG. 1). Further, physiological indicators 112 from sensor system 42 on user 24 may be communicated via at least one of first and second communication links 76, 78. Still further, voice commands 114 from user 24 to drone 26 may be communicated via at least one of first and second communication links 76, 78.

In general, data acquisition unit 96 acquires visual information 116 from camera 88 and physiological indictors 114 received at third communication module 86. Control unit 94 may include a monitoring module 118 (e.g., an artificial intelligence (AI) and machine learning (ML) engine). Visual information 116 may be processed at monitoring module 118 with AI-Machine Learning. For example, a deep learning algorithm may be executed to process visual information 116 and scene depth for obtaining finer details of user 24 in motion. In response, control unit 94 may instruct camera control unit 98 and/or drive control unit 100. Since camera 88 captures visual information 116, this visual information 116 may additionally, or alternatively, include gesture commands. By way of example, user 24 may provide commands to drone 26 by utilizing any variety of movements (e.g., hand, arm, eye, and so forth) that control unit 94 may be configured to interpret as commands for adjusting movement of drone 26, capturing visual information 116, and so forth.

A control algorithm executed at control unit 94 may provide commands to take visual information 116 of the motion of user 24 at predefined locations (e.g., from a front view, side view, top view, and so forth) at periodic intervals or as instructed by user 24 via voice commands 114. Accordingly, control unit 94 may provide motion parameters 120 to drive control unit 100 to adjust a speed and/or position of drone 26 to move drone 26 to a predefined location relative to user 24 using propulsion system 90 to get the desired visual information 116 and/or to get a finer and clearer image. The control algorithm executed at control unit may additionally or alternatively provide camera instructions 122 to camera control unit 98 to focus camera 88 on user 24. In some embodiments, camera instructions 122 may be configured to direct camera 88 along a sight axis 124 (see FIG. 1) between first and second wearable structures 28, 30 such that an auto focus feature of camera 88 is approximately centered on user 24.

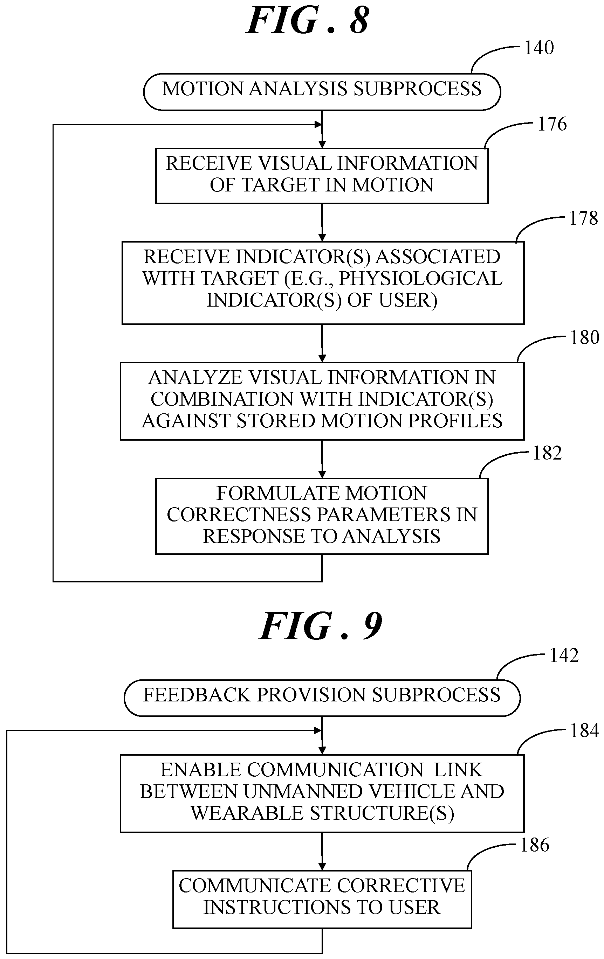

Memory element 106 associated with motion processor 104 may contain one or more databases of preloaded motion profiles 126 of types of motion that may be associated with user 24. For example, motion profiles 126 may include information pertaining to gait or the biomechanics of running, walking, jogging, and so forth from multiple angles of user 24. Motion profiles 126 may additionally include predefined gait rules, proper biomechanics to be followed, past history of the motion of user 24, stored physiological metrics for performance monitoring and so forth. As will be discussed in greater detail in connection with FIG. 8, motion processor 104 may be configured to analyze visual information 116 (e.g., gait information) to determine "gait correctness." In some embodiments, physiological indicators 112 such as heartbeat, speed of user 24, and so forth may be analyzed in combination with visual information 116 to facilitate more meaningful gait analysis.

The term "gait correctness" pertains to motion posture, angle of landing, stride rate, contact time, bounce, and so forth of user 24 in comparison to known sports-specific techniques. Developing a "correct gait" may enhance athletic performance and minimize injury. Further, providing such information to athletes in real time as they traverse a real course can further enhance their development of good motion techniques.

Accordingly, in response to determining gait correctness of user 24 from visual information 116 and physiological indicators 112, motion processor 104 may formulate corrective instructions 128 for user 24. These corrective instructions 128 may encompass any of a wide variety of suggestions such as "lengthen stride," "hold head upright," "don't bend at waist," "take measured breathes," and so forth. As will be discussed in greater detail in connection with FIG. 9, corrective instructions 128 may thereafter be communicated to user 24 from third communication module 86 via at least one of first and second wireless communication links 76, 78.

The terms "engine," "algorithm," "unit," "module," as used herein, refer to logic embodied in hardware or firmware, or to a collection of software instructions written in a programming language and executed by processing unit 84. Processing unit 42 may be a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can include electrical circuitry configured to process computer-executable instructions. Processing unit 84 can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor may also include primarily analog components. For example, some or all of the signal processing algorithms described below may be implemented in analog circuitry or mixed analog and digital circuitry.

First location data 108, second location data 110, physiological indicators 112, voice commands 114, visual information 116, motion parameters 120, camera instructions 122, and corrective instructions 128 are all represented by individual blocks in FIG. 4 for simplicity. This information may be conveyed between the elements of system 20 using various suitable wired and wireless protocols.

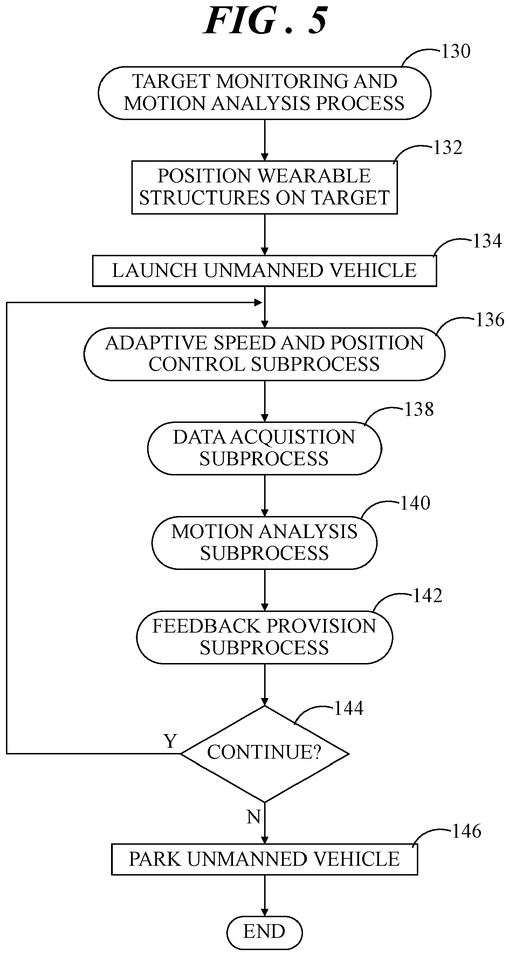

FIG. 5 shows a flowchart of a target monitoring and motion analysis process 130 performed using system 20 (FIG. 1). Target monitoring and motion analysis process 130 provides high level operational blocks and subprocesses associated with intelligently adapting the speed and position of drone 26 relative to user 24 in real time, acquiring and analyzing motion information of user 24, and providing feedback to user 24. Target monitoring and motion analysis process 130 may be performed by drone 26, which may utilizing processing unit 84. For convenience, reference should be made concurrently to FIGs. 1-5 in connection with the ensuing description.

In accordance with an operational block 132 of process 130, first and second wearable structures 28, 30 are positioned on the target. For example, first and second wearable structures, as hearables, are positioned in first and second ears 34, 38 of user 24. Additionally, the elements of sensor system 42 may be suitable positioned, adhered, or otherwise coupled to user 24.

In accordance with an operational block 134 of process 130, the unmanned vehicle (e.g., drone 26) is launched. The launch of drone 26 may occur in response to power up commands by user 24 or by another individual. Drone 26 may be launched from a charging pad or from a launch site near user 24. After drone 26 is launched, and perhaps placed in a hover mode, an adaptive speed and position control subprocess 136, a data acquisition subprocess 138, a motion analysis subprocess 140, and a feedback provision subprocess 142 may be performed.

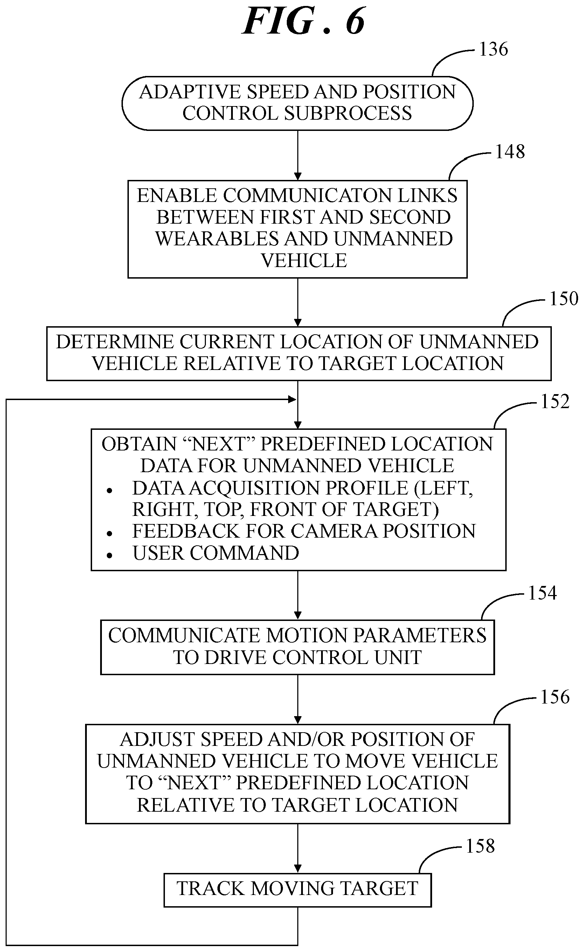

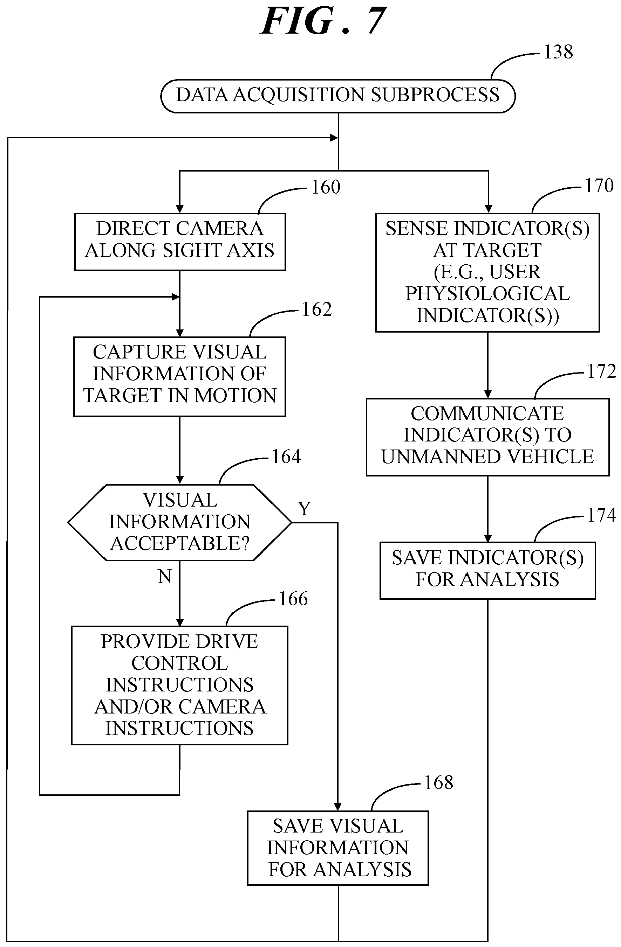

In general, adaptive speed and position control subprocess 136 is executed to determine a current location of drone 26 relative to user 24 and to adjust a speed and position of drone 26 to move drone 26 from the current location to a predefined location relative to user 24. Adaptive speed and position control subprocess 136 will be discussed in connection with the flowchart of FIG. 6. Data acquisition subprocess 138 is executed to receive and save visual information 116 from camera 88 and to receive and save physiological indicators 112 from electronic device 22 positioned on user 24. Data acquisition subprocess 138 will be discussed in connection with the flowchart of FIG. 7. Motion analysis subprocess 140 is executed to determine motion correctness in response to the received visual information 116 and physiological indicators 112. Motion analysis subprocess 140 will be discussed in connection with the flowchart of FIG. 8. Feedback provision subprocess 142 is executed to provide corrective instructions to user 24 in response to the motion analysis. Feedback provision analysis subprocess 142 will be discussed in connection with FIG. 9.

Subprocesses 136, 138, 140, 142 are presented in target monitoring and motion analysis process 130 in sequential order for simplicity. However, it will become apparent in the ensuing discussion, that subprocesses 136, 138, 140, 142 may be performed in any order. Alternatively, some or all of subprocesses 136, 138, 140, 142 may be performed in parallel for enhanced computational efficiency, and to enable the real time exchange of information between processing elements of process unit 84.

At a query block 144, a determination is made as to whether execution of target monitoring and motion analysis process 130 is to continue. By way of example, target monitoring and motion analysis process 130 may be continued for the duration of the user's 24 movement, for some predetermined time period or user travel distance, or until battery monitor circuit 102 determines that battery power of battery 92 is getting low.

When a determination is made at query block 144 that execution of process 130 is to continue, process control loops back to continue execution of adaptive speed and position control subprocess 136, data acquisition subprocess 138, motion analysis subprocess 140, and/or feedback provision subprocess 142. Accordingly, drone 26 is capable of continuously adapting its speed and position in response to the motion of user 24 and/or predefined data collection criteria, acquiring visual information 116 and physiological indicators 112, performing motion analysis, and providing feedback of corrective instructions to user 24 in response to the motion analysis.

When a determination is made at query block 144 that execution of target monitoring and motion analysis process 130 is to be discontinued, drone 26 may be parked on a charging pad or on a landing site. Thereafter, target monitoring and motion analysis process 130 ends.

FIG. 6 shows a flowchart of adaptive speed and position control subprocess 136 of target monitoring and motion analysis process 130 (FIG. 5). Adaptive speed and position control subprocess 136 is performed by drone 26 to continuously enable drone 26 to adapt its speed and position in real time based upon the location of user 24, predefined data acquisition profiles, feedback for camera position, user commands, and so forth. For convenience, reference should be made concurrently to FIGs. 1-4 and 6 in connection with the following description.

At a block 148, first and second wireless communication links 76, 78 are enabled between first and second wearables 28, 30 and the unmanned vehicle (e.g., drone 26). In some embodiments, first, second, and third communication modules 56, 66, 86 of respective first and second wearables 28, 30 and drone 26 are configured to implement a first wireless communication technology to enable first and second wireless communication links 76, 78. The first wireless communication technology may be Bluetooth Classic or Bluetooth Low Energy (BLE) technology. However, other "short-link" wireless technologies, such as Ultra-Wide Band (UWB) for exchanging data between portable devices over short distances with low power consumption may alternatively be implemented. In an example configuration, third communication module 86 of drone 26 may serve as a master device, with first and second communication modules 56, 66 of first and second wearable structures 28, 30 functioning as slave devices. A bonding or pairing procedure may be performed to connect first and second communication modules 56, 66 with third communication module 86.

At a block 150, a current location of the unmanned vehicle (e.g., drone 26) relative to a target location of the target (e.g., user 24) is determined. That is, a target location of user 24 and a current location of drone 26 relative to user 24 may be determined.

By way of example, Bluetooth Core Specification (v5.1) and marketed as Bluetooth 5.1 Direction Finding includes Angle of Arrival (AoA) and Angle of Departure (AoD) features for accurately determining the position of a Bluetooth transmitter in two or three directions. Although Bluetooth 5.1 is mentioned, later versions of Bluetooth 5.x may additionally include AoA and AoD direction finding capability. In an AoA concept, first communication module 56 may broadcast first location data 108 to third communication module 86 at drone 26 via first wireless communication link 76. Processing unit 84 on-board drone 26 measures the arrival angle, θ₁, to determine the location of first wearable structure 28. Similarly, second communication module 66 may broadcast second location data 110 to third communication modules 86 at drone 26 via second wireless communication link 78. Processing unit 84 on-board drone 26 measures the arrival angle, θ₂, to determine the location of second wearable structure 30. From the two arrival angles, θ₁ and θ₂, a target location may be interpolated as a point midway between the individual locations of first and second wearable structures 28, 30. Although AoA is described as one technique, AoD may alternatively be implemented. Further in a UWB application, Time of Flight (ToF) may be utilized to obtain accurate distance/location measurements.

At a block 152, a "next" predefined location data for drone 26 is obtained. The "next" predefined location data may be an initial location of drone 26 relative to user 24, a predefined location based upon a data acquisition profile (e.g., left, right, top, front, or back of user 26, feedback from control unit 94/drive control unit 100 for appropriate camera positioning, user command (e.g., voice command 114 or gesture commands from user 24), or any combination thereof.

At a block 154, motion parameters 120 may be communicated from control unit 94 to drive control unit 100, and at a block 156, drive control unit 100 sends suitable commands to propulsion system 90 to adjust the speed and/or position of drone 26 to move drone 26 to the "next" predefined location relative to the target location. At a block 158, drone 26 thereafter tracks the moving user 24, per the movement of first and second wearable structures 28, 30, maintaining its predefined location relative to user 24. Process flow loops back to block 152 when "next" predefined location data is obtained for drone 26. The execution of adaptive speed and position control subprocess 136 may continue until a determination is made at query block 144 (FIG. 5) that execution of target monitoring and motion analysis process 130 (FIG. 5) is to be discontinued.

Accordingly, the execution of adaptive speed and position control subprocess 136 enables the intelligent positioning of drone 26 relative to user 24 to get the best visual information 116 based on first and second location data 108, 110 from first and second wearable structures 28, 30, control unit 94, and camera control unit 98. Additionally, execution of subprocess 136 enables tracking of user 24 by tracking movement of first and second hearable structures 28, 30.

FIG. 7 shows a flowchart of data acquisition subprocess 138 of target monitoring and motion analysis process 130 (FIG. 5). Data acquisition subprocess 138 may include the concurrent activities of acquiring visual information 116 via camera 88 and acquiring physiological indicators 112 via at least one of first and second communication links 76, 78 from sensor system 42 positioned on user 24. For convenience, reference should be made concurrently to FIGs. 1-4 and 7 in connection with the following description.

With regard to acquiring visual information 116, at a block 160, camera 88 is directed along sight axis 124 which may be approximately centered on user 24 between first and second wearable structures 28, 30. In some embodiments, camera 88 may be suitably positioned by executing adaptive speed and position control subprocess 136 (FIG. 6). At a block 162, visual information 116 of user 24 in motion is captured via camera 88.

At a query block 164, a determination is made as to whether the captured visual information 116 is acceptable. For example, visual information 116 may be processed at control unit 94 for clarity, profile view, or any other factor. When a determination is made at query block 164, that visual information 116 is not acceptable, a block 166 is performed. At block 166, control unit 94 may provide motion parameters 120 to drive control unit 100 to adjust the position and/or speed of drone 26 relative to user 24. Additionally, or alternatively, control unit 94 may provide camera instructions 122 to camera control unit 98 to suitably adjust camera 88 (e.g., focus). Thereafter, program control loops back to block 162 to again capture visual information 116 and determine its acceptability. When a determination is made at query block 166 that visual information is acceptable, subprocess 138 proceeds to a block 168. At block 168, visual information 116 may be communicated to motion processor 104 where it may be saved at least temporarily in, for example, memory 106, for analysis.

With regard to acquiring indicators, at a block 170, indicators such as physiological indicators 112 are sensed at user 24 via sensor system 42. At a block 172, physiological indicators 112 are communicated to drone 26 via at least one of first and second wireless communications links 76, 78. At a block 174, physiological indicators 112 may be communicated to motion processor 104 where the information may be saved at least temporarily in, for example, memory 106, for analysis. Following either of blocks 168 and/or blocks 174, program control loops back to blocks 160 and 170 to continue acquiring visual information 116 and/or physiological indicators 112. The execution of data acquisition subprocess 138 may continue until a determination is made at query block 144 (FIG. 5) that execution of target monitoring and motion analysis process 130 (FIG. 5) is to be discontinued.

Accordingly, the execution of data acquisition subprocess 138 enables the acquisition of visual information 116 via camera 88 and initial assessment of visual information 116 by control unit 94 to acquire the best visual information 116. Additionally, execution of subprocess 138 enables the acquisition of physiological indicators 112 that may enhance motion analysis of user 24.

FIG. 8 shows a flowchart of motion analysis subprocess 140 of target monitoring and motion analysis process 130 (FIG. 5). At subprocess 140, motion processor 104 analyzes the motion of user 24 in real time using the acquired visual information 116 and physiological indicators 112. For convenience, reference should be made concurrently to FIGs. 1-4 and 8 in connection with the following description.

At a block 176, motion processor 104 receives visual information 116 of user 24 in motion. For example, motion processor 104 may access visual information 116 temporarily stored in memory 106. Alternatively, motion processor 104 may receive visual information 116 from control unit 94 or directly from data acquisition unit 96. At a block 178, motion processor 104 receives indicators associated with user 24. These indicators may include physiological indicators 112 communicated via at least one of first and second communication links 76, 78 as discussed previously. Motion processor 104 may access physiological information 112 temporarily stored in memory 106. Alternatively, motion processor 104 may receive physiological indicators 112 from control unit 94 or directly from data acquisition unit 96.

At a block 180, motion processor 104 on-board drone 26 analyzes visual information 116 alone or in combination with physiological indicators 112 against motion profiles 126 stored in memory 106. Motion analysis may entail analyzing the gait requirements based on a selected motion profile (e.g., walking, running, sprinting, and so forth) and/or comparing motion data and physiological indictors 112 with past history (e.g., stored metrics for performance monitoring). Motion processor 104 may analyze visual information 116 against the gait requirements for a selected motion profile to determine "gait correctness." Such motion analysis, sometimes referred to as gait analysis, may be used to optimize athletic performance and/or to identify motions that may cause injury or strain. Further, gait analysis may be used to assess and treat individuals (e.g., user 24) with conditions affecting their ability to walk or run (e.g., cerebral palsy, stroke, and so forth), to identify posture-related or movement-related problems in individuals with injuries, to measure joint positions and velocities, and so forth. Additionally, motion processor 104 may analyze physiological indicators 112 (e.g., heart rate, speed of user 24, force/impact profiles, and so forth) to provide insights into breathing techniques utilized by user 24, pressure/contact distribution, contact area, center of force movement, movement symmetry between sides of the body, and so forth. The execution of motion analysis subprocess 140 may continue until a determination is made at query block 144 (FIG. 5) that execution of target monitoring and motion analysis process 130 (FIG. 5) is to be discontinued.

Thus, gait analysis may be used for sports training, in medical diagnostics to identify pathological gait, in chiropractic and osteopathic scenarios for diagnosing hindrances in gait (e.g., misaligned pelvis or sacrum), and so forth. Further, by studying the gait of non-human species using, for example, only visual information 116, insight may be gained about the mechanics of locomotion of the non-human species.

FIG. 9 shows a flowchart of feedback provision subprocess 142 of target monitoring and motion analysis process 130 (FIG. 5). Feedback provision subprocess 142 may be executed to provide feedback to user 24 of their motion so that user 24 may take corrective measures. In the instance that the target is an inanimate object or a non-human animal, feedback provision subprocess 142 may not be executed. For convenience, reference should be made concurrently to FIGs. 1-4 and 9 in connection with the following description.

At a block 184, a communication link is enabled between the unmanned vehicle (e.g., drone 26) and at least one of first and second wearable structures 28, 30 of electronic device 22 positioned on the target (e.g., user 24). Communication between third communication module 86 of drone 26 and first and second communication modules 56, 66 of respective first and second wearable structures 28, 30 may be enabled utilizing the first communication technology (e.g., BLE). Thus, communication may have been previously enabled via at least one of first and second wireless communication links 76, 78.

At a block 186, corrective instructions 128 are communicated to user 24. Corrective instructions 128 may be audible instructions broadcast to user 24 via one or both speakers 62, 72 of respective first and second wearable structures 28, 30. Thus, user 24 can be provided with meaningful feedback on, for example, gait technique, breathing technique and so forth in real time in order to take corrective measures while still in motion on real terrain. The execution of feedback subprocess 138 may continue until a determination is made at query block 144 (FIG. 5) that execution of target monitoring and motion analysis process 130 (FIG. 5) is to be discontinued.

Thus, execution of the various processes described herein enable autonomous real time positioning of an unmanned vehicle relative to a target to be monitored, data acquisition of visual information motion of the target and, in some embodiments, physiological indicators of the target, motion analysis of the motion of the target based on the visual information and the physiological indicators, and feedback to the target regarding the motion analysis. It should be understood that certain ones of the process blocks depicted in FIGs. 5-9 may be performed in parallel with each other or with performing other processes. In addition, the particular ordering of the process blocks depicted in FIGs. 5-9 may be modified while achieving substantially the same result. Accordingly, such modifications are intended to be included within the scope of the inventive subject matter.

The above discussion focused primarily on monitoring and motion analysis of a target, primarily a human user, so that the user may take corrective action as needed. However, the system may be adapted for other applications. For example, motion data may additionally or alternatively be reviewed in non-real time for judging criteria, for post run analysis, and so forth. The motion data may be utilized for golf technique analysis or other sports. Further, hearable structures and sensor system for collecting physiological indicators and tracking via a drone may be implemented for the elderly or a vulnerable user as a safeguard, such as fall detection. Still further, the hearable structures and drone may be used with a first responder. In such a scenario, the first responder may provide voice commands to the drone for monitoring a crowd and/or for location finding within the crowd, for monitoring a fire, as a lifeguard for monitoring swimmers on a beach or in a pool, and so forth. In another possible application, the hearable structures and drone may be used for alignment of one or more drone cameras for precision measurement in building construction, heritage building structural safety monitoring, and the like.

Embodiments described herein entail a system and methodology for monitoring motion of a target, such as a human user. More particularly, the system and methodology entail real time autonomous positioning and navigation of an unmanned vehicle relative to the moving target. The unmanned vehicle and an electronic device positioned on the target communicate to locate the target and position the unmanned vehicle relative to the target. The unmanned vehicle includes a sensor system (e.g., a camera) for detecting motion of the moving target. In some embodiments, the electronic device positioned on the target also includes a sensor system (e.g., motion/pressure sensors, vitals monitors, and so forth) configured to detect physiological indicators of the target. The unmanned vehicle includes a processing unit configured to adjust the position of the unmanned vehicle relative to the moving target, control the on-board sensor system (e.g., camera), receive visual information of the motion of the target, receive the physiological indicators of the target, analyze the motion of the target (e.g., human user) based on the visual information alone or in combination with and the physiological indicators, and provide real time feedback to the human user regarding the motion analysis. In some embodiments, a human user can provide voice commands via the electronic device to control the unmanned vehicle.

The disclosure extends to the following clauses:
A The system of any of claims 1 to 9 wherein: the first, second, and third communication modules are configured to implement a first wireless communication technology to enable the first and second wireless communication links; and the first and second wearable structures are configured to implement a second wireless communication technology that differs from the first wireless communication technology to communicate with one another.
B The system of Clause A wherein the electronic device further comprises a target sensor system positioned on the target, the target sensor system being configured to sense at least one indicator associated with the target and communicate the at least one indicator to one of the first and second communication modules of the first and second wearable structures using a third wireless communication link and implementing the second wireless communication technology, wherein the at least one indicator is communicated to the processing unit via at least one of the first and second wireless communication links implementing the first wireless communication technology.
C The system of any of claims 1 to 9, or clause A or B wherein the target comprises a user, and: the first wearable structure includes at least a first portion configured to be disposed within a first ear of the user; and the second wearable structure includes at least a second portion configured to be disposed within a second ear of the user.
D The system of any of claims 1 to 9, or clauses A, B or C, wherein the target comprises a user, and the electronic device further comprises a microphone positioned on the user for input of voice commands from the user, wherein the voice commands are communicated via at least one of the first and second wireless communication links to the unmanned vehicle.

This disclosure is intended to explain how to fashion and use various embodiments in accordance with the invention rather than to limit the true, intended, and fair scope and spirit thereof. The foregoing description is not intended to be exhaustive or to limit the invention to the precise form disclosed. Modifications or variations are possible in light of the above teachings. The embodiment(s) was chosen and described to provide the best illustration of the principles of the invention and its practical application, and to enable one of ordinary skill in the art to utilize the invention in various embodiments and with various modifications as are suited to the particular use contemplated. All such modifications and variations are within the scope of the invention as determined by the appended claims, as may be amended during the pendency of this application for patent, and all equivalents thereof, when interpreted in accordance with the breadth to which they are fairly, legally, and equitably entitled.

## Claims

1. A system comprising:
an electronic device comprising:
a first wearable structure configured to be positioned on a target, the first wearable structure including a first communication module; and
a second wearable structure configured to be positioned on the target, the second wearable structure being physically displaced away from the first wearable structure, the second wearable structure including a second communication module; and
an unmanned vehicle for monitoring motion of the target, the unmanned vehicle comprising:
a third communication module, wherein the first and third communication modules are configured to enable a first wireless communication link between the first wearable structure and the unmanned vehicle, and the second and third communication modules are configured to enable a second wireless communication link between the second wearable structure and the unmanned vehicle;
a processing unit configured to determine a current location of the unmanned vehicle relative to the target in response to the first and second wireless communication links; and
a drive control unit in communication with the processing unit and configured to adjust a speed and a position of the unmanned vehicle to move the unmanned vehicle from the current location to a predefined location relative to the target.

2. The system of claim 1 wherein:
at least one of the first and third communication modules is configured to communicate first location data via the first wireless communication link for receipt at the other of the first and third communication modules;
at least one of the second and third communication modules is configured to communicate second location data via the second wireless communication link for receipt at the other of the second and third communication modules; and
the processing unit is configured to determine a target location of the target based on the first and second location data and communicate motion parameters to the drive control unit in response to the target location, wherein the motion parameters enable the drive control unit to adjust the speed and the position of the unmanned vehicle to move the unmanned vehicle to the predefined location relative to the target location.

3. The system of claim 1 or 2 wherein the predefined location is a first predefined location, the drive control unit is further configured to adjust the speed and the position of the unmanned vehicle to move the unmanned vehicle from the first predefined location to a second predefined location.

4. The system of any preceding claim wherein the unmanned vehicle further comprises a sensor system configured to detect motion of the target and provide motion information of the target to the processing unit.

5. The system of claim 4 wherein the processing unit is further configured to update the motion parameters for enabling the drive control unit to adjust the speed and the position of the unmanned vehicle in response to the motion information.

6. The system of claim 4 or 5 wherein the sensor system comprises a camera, and the motion information comprises visual information of the target in motion.

7. The system of claim 6 wherein the processing unit is further configured to direct the camera along a sight axis between the first and second wearable structures for capturing the visual information.

8. The system of any of claims 4 to 7 wherein the target is a user, and:
the processing unit is further configured to determine gait correctness of the user from the motion information; and
the third communication module is configured to communicate corrective instructions to the user in response to the gait correctness via at least one of the first and second wireless communication links.

9. The system of claim 8 wherein the electronic device further comprises a user sensor system positioned on the user and configured to sense at least one physiological indicator of the user, wherein the at least one physiological indicator is communicated to the processing unit via at least one of the first and second wireless communication links, and the processing unit is further configured to determine the gait correctness of the user from the at least one physiological indicator in combination with the gait information.

10. A method utilizing an unmanned vehicle for monitoring motion of a target comprising:
positioning first and second wearable structures of an electronic device on the target, the first and second wearable structures being physically displaced away from one another, the first wearable structure including a first communication module, and the second wearable structure including a second communication module;
enabling a first wireless communication link between the first communication module of the first wearable structure and a third communication module on-board the unmanned vehicle;
enabling a second wireless communication link between the second communication module of the second wearable structure and the third communication module;
determining a current location of the unmanned vehicle relative to the target in response to the first and second wireless communication links; and
adjusting a speed and a position of the unmanned vehicle to move the unmanned vehicle from the current location to a predefined location relative to the target.

11. The method of claim 10 further comprising:
the enabling the first wireless communication link comprises communicating first location data via the first wireless communication link;
the enabling the second wireless communication link comprises communicating second location data via the second wireless communication link; and
determining, at a processing unit on-board the unmanned vehicle, a target location of the target based on the first and second location data, wherein the adjusting operation utilizes the target location to move the unmanned vehicle to the predefined location relative to the target location.

12. The method of claim 10 or 11 wherein the predefined location is a first predefined location, and the method further comprises adjusting the speed and the position of the unmanned vehicle to move the unmanned vehicle from the first predefined location to a second predefined location.

13. The method of any of claims 10 to 12 further comprising obtaining motion information of the target, at a sensor system on-board the unmanned vehicle, wherein the adjusting operation utilizes the motion information to adjust the speed and the position of the unmanned vehicle, and optionally wherein the sensor system comprises a camera, the motion information comprises visual information of the target in motion, and the method further comprises directing the camera along a sight axis between the first and second wearable structures for capturing the visual information.

14. The method of claim 13 wherein the target comprises a user, and the method further comprises:
sensing at least one physiological indicator of the user at a sensor system positioned on the user;
communicating the at least one physiological indicator to a processing unit on-board the unmanned vehicle via at least one of the first and second wireless communications links;
determining gait correctness of the user from the at least one physiological indicator in combination with the motion information at the processing unit on-board the unmanned vehicle; and
communicating corrective instructions to the user in response to the gait correctness from the third communication module via at least one of the first and second wireless communication links.

15. The method of any of claims 10 to 14 wherein the target comprises a user, and the method further comprises:
receiving voice commands from the user at a microphone on-board the electronic device; and
communicating the voice commands to the unmanned vehicle via at least one of the first and second wireless communication links to the unmanned vehicle.
